# EUROPÄISCHE PATENTSCHRIFT

(11) **EP 0 945 415 B1**
(45) Veröffentlichungstag und Bekanntmachung des Hinweises auf die Patenterteilung: **07.11.2001**
(21) Anmeldenummer: 99105125.1
(22) Anmeldetag: 26.03.1999
(51) Int. Cl.: C07C 11/06, C07C 7/163, C07C 41/06

(54) **Verfahren zur Herstellung von Olefinen**
Process for the preparation of olefins
Procédé pour la préparation d'oléfines

(30) Priorität: 27.03.1998 DE 19813720
(43) Veröffentlichungstag der Anmeldung: 29.09.1999
(73) Patentinhaber: BASF AKTIENGESELLSCHAFT, 67056 Ludwigshafen (DE)
(72) Erfinder: Schwab, Peter, Dr., 67098 Bad Dürkheim (DE); Schulz, Ralf, Dr., 67346 Speyer (DE); Schulz, Michael, Dr., 67067 Ludwigshafen (DE); Breitscheidel, Boris, Dr., 67117 Limburgerhof (DE); Meyer, Gerald, Dr., 67071 Ludwigshafen (DE)
(74) Vertreter: Isenbruck, Günter, Dr.

(56) Entgegenhaltungen:
- EP-A- 0 742 195
- EP-A- 0 832 867
- WO-A-97/32838

## Beschreibung

Die Erfindung betrifft ein Verfahren zur Herstellung von Olefinen, insbesondere Propen aus Steamcracker- oder Raffinerie-C₄-Strömen in einem gekoppelten Metathese-Verfahren.

Steamcracker stellen die Hauptquelle für petrochemische Basischemikalien, wie Ethen, Propen, C₄-Olefine und höhere Kohlenwasserstoffe dar. Beim Crackprozeß ist es notwendig, große Energiemengen bei hohen Temperaturen in einer Zeitspanne zu übertragen, die einerseits zwar ausreicht, um die Spaltung durchzuführen, andererseits aber eine Weiterreaktion der Spaltprodukte nicht zuläßt. Bei der Spaltung von Kohlenwasserstoffen wird die Ausbeute an Ethen und Propen daher im wesentlichen durch die
- Art der eingesetzten Kohlenwasserstoffe (Naphtha, Ethan, LPG, Gasöl, o.ä.),
- Spalttemperatur
- Verweilzeit
- und Partialdrücke der Kohlenwasserstoffe
bestimmt.

Die höchste Ausbeute an Ethen und Propen wird bei Spalttemperaturen zwischen 800 und 850°C und Verweilzeiten von 0,2 bis 0,5 s erzielt. Hauptprodukt ist in diesem Bereich stets Ethen, wobei das C₃/C₂-Austragsverhältnis von ca. 0,5 bis 0,7 durch Variation der Crack-Bedingungen nur in geringem Ausmaß erhöht werden kann. Weltweit steigt der Bedarf an Propen jedoch rascher an als der an Ethen. Dies hat u.a. zur Folge, daß Verfahren zur Downstream-Verwertung der höheren, beim Crackprozeß gebildeten Kohlenwasserstoffe, wie C₄, im Hinblick Werkzeug hierfür liefert die Metathese von Raffinat II bzw. 2-Buten mit Ethen zu Propen. Um die hierzu erforderliche Zusammensetzung des C₄-Feedstocks zu gewährleisten, müssen der Roh-C₄-Strom gereinigt sowie störende Nebenkomponenten effizient abgetrennt werden.

Verschiedene Kombinationsverfahren zur Herstellung von Propen, die einen oder mehrere Metatheseschritte beinhalten, sind bekannt. In der US 5,300,718 ist ein Verfahren beschrieben, bei dem ein gemischter C₄-Strom, der 1-Buten, 2-Buten, Isobuten, Butadien und Butane enthält, in eine Veretherungszone eingeführt wird, in der das Isobuten mit einem Alkohol in einen Ether überführt wird, der aus dem C₄-Strom abgetrennt wird. Der Isobuten-abgereicherte Strom wird sodann mit einem Butadien-selektiven Lösungsmittel kontaktiert, um Butadien zu entfernen. Der so Butadien-abgereicherte Raffinatstrom wird mit einem adsorbierenden Material kontaktiert, um verbliebene Alkohole und Ether zu entfernen und einen deoxygenierten Strom zu bilden, der nachfolgend an einem Olefinisomerisierungskatalysator zur Erhöhung der Konzentration an 2-Buten und zur Verminderung der Konzentration an 1-Buten isomerisiert wird. Der erhaltene Isomerisatstrom wird zusammen mit Ethen in Gegenwart eines Disproportionierungskatalysators einer Metathese zur Bildung von Propen unterworfen.

In der EP-A-0 742 195 ist ein Verfahren zur Umwandlung eines C₄-Schnitts in Ether und Propen beschrieben. Dazu wird ein Roh-C₄-Strom einer selektiven Hydrierung der Butadiene und acetylenischen Verunreinigungen mit gleichzeitiger Isomerisierung von 1-Buten zu 2-Buten unterworfen. Nachfolgend wird das im Reaktionsaustrag enthaltene Isobuten mit einem Alkohol in Gegenwart eines sauren Veretherungskatalysators umgesetzt und als Ether enfernt. Es schließt sich eine Abtrennung von Oxygenat-Verunreinigungen an, worauf der an 2-Buten reiche Strom mit Ethen in Gegenwart eines Metathese-Katalysators zu Propen umgesetzt wird.

In den bekannten Verfahren erfolgt die Herstellung von Propen unter Zudosierung von mindestens equimolaren Mengen an Ethen.

Aufgabe der vorliegenden Erfindung ist die Bereitstellung eines Verfahrens zur Herstellung von Olefinen, insbesondere Propen als Koppelprodukt ausgehend von Steamcracker- oder Raffinerie-C₄-Strömen, wobei die Nachteile der bekannten Verfahren vermieden werden sollen.

Die Aufgabe wird erfindungsgemäß gelöst durch ein Verfahren zur Herstellung von Olefinen, insbesondere Propen aus Steamcracker- oder Raffinerie-C₄-Strömen durch
A) gegebenenfalls Extraktivdestillation von Butadien aus dem C₄-Strom mit einem Butadien-selektiven Lösungsmittel, um einen weitgehend Butadienabgereicherten C₄-Strom zur erhalten,
B) Selektivhydrierung von Butadienen und acetylenischen Verunreinigungen im Steamcracker- oder Raffinerie-C₄-Strom oder dem Strom aus Schritt A), mit gleichzeitiger oder nachgeschalteter zumindest teilweiser Isomerisierung von 1-Buten zu 2-Buten, um einen C₄-Strom zu erhalten, der n-Butene und i-Buten enthält und im wesentlichen frei von Butadienen und acetylenischen Verunreinigungen ist,
C) Entfernung von i-Buten aus dem in Schritt B) erhaltenen C₄-Strom durch Umsetzung mit einem Alkohol zur Bildung eines Ethers, der abgetrennt und gegebenenfalls zur Gewinnung von reinem Isobuten rückgespalten wird, um einen C₄-Strom zu erhalten, der n-Butene und gegebenenfalls Oxygenat-Verunreinigungen enthält und aus dem bei der Abtrennung des Ethers auch niedriger und höher siedende Verbindungen destillativ entfernt werden können,
D) Abtrennen von Oxygenat-Verunreinigungen aus dem in Schritt C) erhaltenen C₄-Strom mit Adsorbermaterialien,
E) zweistufige Metathese der Butene im in Schritt D) erhaltenen C₄-Strom durch
   a) Umsetzung von im C₄-Strom enthaltenem 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
   b) anschließendes Trennen des gebildeten Propens und 2-Pentens und der nicht umgesetzten Butene und gegebenenfalls zumindest teilweise Ausschleusen einer oder mehrerer dieser Verbindungen
   c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
   d) anschließendes Trennen des gebildeten Propens und 1-Butens,
   e) gegebenenfalls Ausschleusen eines reinen 1-Buten-Stroms und/oder gegebenenfalls Isomerisieren des gebildeten 1-Butens zu 2-Buten in Gegenwart eines Isomerisierungskatalysators und anschließendes Rückführen des nicht ausgeschleusten 1-Butens und gegebenenfalls gebildeten 2-Butens gemeinsam mit einem Teil der in Schritt a) nicht umgesetzten C₄-Fraktion in Schritt a),
   f) Gewinnen des in den Schritten b) und d) abgetrennten Propens,
wobei die Trennung in Schritt d) durch Überführen des umgesetzten Gemisches in Schritt b) erfolgen kann, wobei dann in Schritt e) die in Schritt b) abgetrennte, nicht umgesetzte C₄-Fraktion gegebenenfalls zumindest teilweise ausgeschleust und/oder gegebenenfalls das in dieser C₄-Fraktion enthaltene 1-Buten in Gegenwart eines Isomerisierungskatalysators zumindest teilweise zu 2-Buten isomerisiert wird und das erhaltene Gemisch anschließend in Schritt a) zurückgeführt wird.

Das Verfahren kann so gesteuert werden, daß wahlweise als Produkte eine oder mehrere der Verbindungen Propen, 1-Buten, 2-Penten, 3-Hexen in Schritt E, Butadien in Schritt A und Isobuten oder MTBE in Schritt C erhalten werden.

Die in Schritt E durchgeführten Metathesereaktionen sind in der prioritätsälteren, nicht vorveröffentlichten DE-A-196 40 026 beschrieben:

Die Olefinmetathese (Disproportionierung) beschreibt in ihrer einfachsten Form die reversible, metallkatalysierte Umalkylidenierung von Olefinen durch Bruch und Neuformierung von C=C-Doppelbindungen. Dabei werden beispielsweise Olefine der Formeln R¹-CH=CH-R² und R³-CH=CH-R⁴ reversibel zu Olefinen der Formeln R¹-CH=CH-R³ und R²-CH=CH-R⁴ umgesetzt. Bei der Metathese acyclischer Olefine unterscheidet man zwischen Selbstmetathese, bei der ein Olefin in ein Gemisch zweier Olefine unterschiedlicher molarer Massen übergeht, und Kreuz- oder Co-Metathese, bei der zwei unterschiedliche Olefine reagieren. Ein Beispiel für eine Selbstmetathese ist die Umsetzung von zwei Molekülen Propen zu Ethen und 2-Buten, wie sie beispielsweise nach dem Phillips-Triolefin-Verfahren ausgerührt wird, siehe Hydrocarbon Processing, Band 46, Nr. 11, November 1967, S. 232. Ein Beispiel für eine Kreuz-Metathese ist die Umsetzung von Propen und 1-Buten zu Ethen und 2-Penten. Ist einer der Reaktionspartner Ethen, so spricht man üblicherweise von einer Ethenolyse.

Die Metathesereaktionen erfolgen in Gegenwart von Katalysatoren. Hierzu eignen sich prinzipiell homogene und heterogene Übergangsmetallverbindungen, insbesondere solche der VI bis VIII Nebengruppe des Periodensystems der Elemente, wie auch homogene und heterogene Katalysatorsysteme, in denen diese Verbindungen enthalten sind.

Aus EP-A1-0 691 318 ist beispielsweise ein Olefinmetatheseverfahren bekannt, bei dem C₅-Olefine und Ethen in Gegenwart eines Katalysators zu gemischten C₄-Olefinen und Propen umgesetzt werden. So wird 2-Methyl-2-buten mit Ethen zu Isobuten und Propen umgesetzt. Ein Gemisch aus 2-Pentenen und 2-Methyl-2-buten wird zu einem Gemisch aus 1-Buten, Isobuten und Propen umgesetzt.

Das erfindungsgemäße Verfahren beinhaltet in Schritt E) 2 Metatheseschritte. Im ersten Schritt werden im C₄-Strom enthaltenes 1-Buten und 2-Buten zu Propen und 2-Penten umgesetzt. In einem zweiten Schritt wird dann 2-Penten mit Ethen zu 1-Buten und Propen umgesetzt. Das 1-Buten wird gemäß einer Ausführungsform der Erfindung dabei gegebenenfalls zumindest teilweise in Gegenwart eines Isomerisierungskatalysators zu 2-Buten isomerisiert und das resultierende Gemisch aus 1-Buten und 2-Buten in den ersten Reaktionsschritt zurückgeführt. Hierdurch wie auch durch Regelung des Umsatzes am Isomerisierungskatalysator kann ein optimales Mengenverhältnis von 1-Buten zu 2-Buten am Eingang des ersten Metathesereaktors eingestellt werden, um beispielsweise eine maximale Ausbeute an Propen zu erreichen. Durch die zweistufige Verfahrensweise mit Kreuzmetathese und Ethenolyse kann der Rohstoffbedarf an Ethen und C₄-Olefinen gegenüber einstufigen Ethenolyseverfahren, wie sie beispielsweise in US 3,660,506 und EP-A-0 273 817 beschrieben sind, um etwa 5 bis 15% verringert werden.

Beide Metatheseschritte können als Reaktivdestillation ausgerührt werden, wie sie nachstehend beschrieben ist.

Nachfolgend wird die Vorbehandlung von C₄-Strömen beschrieben, um zu einem in der Metathese einsetzbaren C₄-Strom zu gelangen.

Der C₄-Strom kann beispielsweise aus einem Cracker, insbesondere Steamcracker, oder einer Raffination stammen. Der C₄-Strom enthält dabei in der Regel neben ungesättigten auch gesättigte C₄-Kohlenwasserstoffe. Der Cracker kann wie eingangs beschrieben betrieben werden.

Die Weiterbehandlung des C₄-Stroms erfolgt durch
1. Selektivhydrierung/Butadienextraktivdestillation von Roh-C₄-Schnitt zur Entfernung von 1,3-Butadien, 1,2-Butadien, 1-Butin (Ethylacetylen) und Butenin (Vinylacetylen),
2. i-Buten-Abtrennung aus hieraus gebildetem sogenanntem Raffinat I via Veretherung mit Alkoholen sowie
3. Reinigung des hierbei erhaltenen Raffinat II-Stroms an Adsorbermaterialien zur Abtrennung von Oxygenates, Schwefelverbindungen, Wasser, Chloriden und sonstigen den Nachfolgeschritt störenden Nebenkomponenten, sowie die vorstehend beschriebene
4. Metathese von Raffinat II bzw. 2-Buten mit Ethen zu Propen.

### Selektivhydrierung von Roh-C₄ (Schritt B)

Alkine, Alkinene und Alkadiene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark.
Der C₄-Strom eines Steamcrackers enthält mit bis zu 70 Gew.-% einen sehr hohen Anteil mehrfach ungesättigter Verbindungen wie 1,3-Butadien, 1,2-Butadien, 1-Butin (Ethylacetylen) und Butenin (Vinylacetylen). Je nach vorhandener Downstream-Verarbeitung werden die mehrfach ungesättigten Verbindungen entweder extrahiert (Butadien-Extraktivdestillation) oder selektiv hydriert. Im erstgenannten Fall beträgt der Restgehalt mehrfach ungesättigter Verbindungen typischerweise 0,1-4 Gew.-%, im letztgenannten Fall typischerweise 0,001-0,3 Gew.-%. Da die Restmengen an mehrfach ungesättigten Verbindungen ebenfalls bei der Weiterverarbeitung stören, ist eine weitere Absicherung durch Selektivhydrierung auf Werte < 10 ppm erforderlich. Um die Wertprodukte Butene zu erhalten, ist die Überhydrierung zu den Butanen so gering wie möglich zu halten. Ähnliche Aufgabenstellungen liegen auch zur Behandlung von C₄-Einsatzstoffen aus FCC-Verfahren ("Fluid Catalytic Cracking") vor.

Geeignete Verfahren sind beispielsweise in H. Lauer, Eröl und Kohle - Erdgas - Petrochemie, 36 (Juni 1983) 249-251 (Kalthydrierung von ungesättigten Kohlenwasserstoffströmen an Edelmetallkatalysatoren in einem Röhenreaktor) sowie EP-A-0 780 155 beschrieben.

### Extraktion von Butadien aus Roh-C₄ (Schritt A)

Sämtliche geeigneten modernen Verfahren zur Butadien-Isolierung basieren auf dem physikalischen Prinzip der Extraktivdestillation. Durch Zusatz selektiver organischer Lösungsmittel wird die Flüchtigkeit spezieller Komponenten eines Gemisches, in diesem Fall Butadien, erniedrigt. Diese bleiben daher mit dem Lösungsmittel im Sumpf der Destillationskolonne, während die destillativ zuvor nicht abtrennbaren Begleitsubstanzen über Kopf entfernt werden können. Als Lösungsmittel für die Extraktivdestillation kommen hauptsächlich Aceton, Furfurol, Acetonitril, Dimethylacetamid, Dimethylformamid (DMF) und N-Methylpyrrolidon (NMP) zur Anwendung. Extraktivdestillationen eignen sich besonders für butadienreiche C₄-Crackschnitte mit einem relativ hohen Anteil an Alkinen, u.a. Methyl-, Ethyl- und Vinylacetylen sowie Methylallen. In modernen Verfahren mit Lösungsmitteln wie Dimethylformamid (Nippon Zeon, Leuna), Dimethylacetamid (UCC) oder N-Methylpyrrolidon (BASF, ABB Lummus Crest) ist die Alkin-Abtrennung ein Teilschritt im Verfahrensablauf. Zu den älteren, besonders in USA entwikelten und genutzten Verfahren mit Lösungsmitteln wie Aceton, Furfurol (Phillips Petroleum) oder Acetonitril (Shell, UOP, ARCO) war eine vorgeschaltete partielle Hydrierung notwendig, um eine störende Harzbildung zu vermeiden.
Das vereinfachte Prinzip einer Lösungsmittel-Extraktion aus Roh-C₄-Schnitt kann wie folgt dargestellt werden: Der vollständig verdampfte C₄-Schnitt wird einer Extraktionskolonne am unteren Ende zugeführt. Das Lösungsmittel (DMF, NMP) fließt von oben dem Gasgemisch entgegen und belädt sich auf dem Weg nach unten mit besser löslichem Butadien und geringen Mengen an Butenen. Am unteren Ende der Extraktionskolonne wird ein Teil des gewonnenen Rein-Butadiens zugeführt, um die Butene weitestgehend auszutreiben. Die Butene verlassen die Trennsäule am Kopf. In einer als Ausgaser bezeichneten weiteren Kolonne wird das Butadien durch Auskochen vom Lösungsmittel befreit und anschließend reindestilliert. Im bevorzugten NMP-Verfahren wird Reinbutadien in 99,8 % Reinheit erhalten. Die Butadien-Ausbeute beträgt 96 % bezogen auf den ursprünglichen Gehalt im Roh-C₄-Schnitt.

Erfindungsgemäß wird der Reaktionsaustrag einer Butadien-Extraktivdestillation in die zweite Stufe einer Selektivhydrierung eingespeist, um den Butadien-Restgehalt auf Werte von < 10 ppm zu reduzieren.

Der nach Abtrennung von Butadien verbleibende C₄-Strom wird als C₄-Raffinat oder Raffinat I bezeichnet und enthält in der Hauptsache die Komponenten i-Buten, 1-Buten, 2-Butene sowie n- und i-Butan.

### Abtrennung und Gewinnung von i-Buten aus Raffinat I (Schritt C)

Bei der weiteren Auftrennung des C₄-Stroms wird nachfolgend i-Buten extraktiv durch Umsetzung mit Alkoholen an sauren Ionenaustauschern entfernt. Hierzu werden vorzugsweise Methanol (ergibt MTBE) oder i-Butanol (ergibt IBTBE) eingesetzt.

Die Herstellung von MTBE aus Methanol und i-Buten erfolgt bei 30-100°C und leichtem Überdruck in der Flüssigphase an sauren Ionenaustauschern. Man arbeitet entweder in zwei Reaktoren oder in einem zweistufigen Schachtreaktor, um einen nahezu vollständigen i-Buten-Umsatz (>99%) zu erzielen. Die druckabhängige Azeotropbildung zwischen Methanol und MTBE erfordert zur Reindarstellung von MTBE eine mehrstufige Druckdestillation oder wird nach neuerer Technologie (Erdölchemie/Bayer) durch Methanol-Adsorption an Adsorberharzen erreicht. Alle anderen Komponenten der C₄-Fraktion bleiben unverändert. Geringe Anteile von Diolefinen und Acetylenen bewirken durch Polymerbildung eine Verkürzung der Lebensdauer des Ionenaustauschers. Neue Entwicklungen haben zu einem bifunktionelle Pd-enthaltenden Ionenaustauscher geführt, bei dem in Gegenwart kleiner Mengen Wasserstoff nur Diolefine und Acetylene hydriert werden. Die Veretherung des i-Butens bleibt hiervon unbeeinflußt. MTBE dient in erster Linie zur Octanzahl-Erhöhung von Fahrbenzin.

Die Veretherung von i-Buten mit i-Butanol zu IBTBE erfolgt gemäß EP-B-0 003 305 und EP-B-0 015 513 an saurem Ionenaustauscher. Vorzugsweise findet die Umsetzung in einer Reaktorkaskade aus drei annähernd adiabtisch betriebenen Festbettreaktoren mit Zwischenkühlungen bei Drücken von 8-20 bar statt. Der Hauptumsatz erfolgt im ersten Reaktor (80-90%), die Vervollständigung des Umsatzes bis zum Gleichgewichtsumsatz, der bei hohen Drücken und niedrigen Temperaturen bei 98-99% liegt, wird in den restlichen Stufen erreicht. In der anschließenden Kolonne wird Raffinat II als Seitenstrom abgezogen, wobei nicht umgesetztes i-Buten verbleibt (Spez. < 3%). IBTBE und höher siedende Stoffe werden im Sumpf abgezogen.

IBTBE und MTBE können an sauren Oxiden in der Gasphase bei 150-300°C zur Reingewinnung von i-Buten rückgespalten werden. Hierzu wird der z.B. vorwiegend aus IBTBE bestehende Sumpfstrom verdampft und an saurem Katalysator zu i-Buten und i-Butanol rückgespalten. In der anschließenden Trennsequenz wird reines i-Buten als Kopfprodukt abgezogen.

Der Vorteil des IBTBE-Verfahrens gegenüber dem MTBE-Verfahren für das erfindungsgemäße Verfahren besteht darin, daß keine leichtflüchtigen Oxygenates wie Dimethylether gebildet werden, welche in der destillativen Aufarbeitung der Veretherung nicht hinreichend abgetrennt werden können und für die nachfolgenden Metathesereaktion starke Katalysatorgifte darstellen.

### Feedreinigung des Raffinat II-Stroms an Adsorbermaterialien (Schritt D)

Zur Verbesserung der Standzeit der eingesetzten Katalysatoren für den nachfolgenden Metatheseschritt ist der Einsatz einer Feed-Reinigung (Guard Bed) zur Abtrennung von Katalysatorgiften, wie Wasser, Oxygenates, Schwefel oder Schwefelverbindungen bzw. organischen Halogeniden erforderlich.

Geeignete Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974).

Die beschriebene Verfahrenssequenz zur Verwertung von C₄-Schnitt aus Steamcrackern oder FCC-Crackern in Richtung Propen ermöglicht im Vergleich zu herkömmlichen Verfahren eine höhere Wirtschaftlichkeit in bezug auf die Nutzung der Einsatzstoffe Roh-C₄ und Ethen.

Nachfolgend werden die einzelnen Verfahrensschritte der bevorzugten Ausführungsform näher beschrieben.

### Selektivhydrierung von Roh-C₄-Schnitt (Schritt B)

Aus der aus einem Steamcracker oder einer Raffinerie stammenden Roh-C₄-Fraktion wird zunächst Butadien (1,2- und 1,3-Butadien) sowie im C₄ enthaltene Alkine oder Alkenine in einem zweistufigen Verfahren selektivhydriert. Der aus der Raffinerie stammende C₄-Strom kann gemäß einer Ausführungsform auch direkt in den zweiten Schritt der Selektivhydrierung eingespeist werden. Der erste Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 40 bis 80°C und einem Druck von 10 bis 30 bar, einem Molverhältnis von Wasserstoff zu Butadien von 10 bis 50 und einer Volumengeschwindigkeit ("Liquid Hourly Space Velocity" LHSV) von bis 15 m³ Frischfeed pro m³ Katalysator pro Stunde und einem Verhältnis von Recycle zu Zustrom von 5 bis 20.
Der zweite Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 50 bis 90°C und einem Druck von 10 bis 30 bar, einem Molverhältnis von Wasserstoff zu Butadien von 1,0 bis 10 und einer Volumengeschwindigkeit ("Liquid Hourly Space Velocity" LHSV) von 5 bis 20 m³ Frischfeed pro m³ Katalysator pro Stunde und einem Verhältnis von Recycle zu Zustrom von 0 bis 15.

Die Hydrierung wird vorzugsweise unter low- bis medium-isom-Bedingungen durchgeführt, unter denen es zu einer geringen bis mittleren Butenisomerisierung, insbesondere von 1-Buten zu 2-Buten, kommt. Der Restgehalt an Butadien kann je nach Hydrierschärfe 1 bis 100 ppm betragen.

Der so erhaltene Reaktionsaustrag wird als Raffinat I bezeichnet und enthält neben i-Buten und n/i-Butan 1-Buten und 2-Buten in einem fiir die Metathese optimierten Molverhältnis.

### alternativ: Abtrennung von Butadien aus Roh-C₄-Schnitt via Extraktivdestillation Schritt A)

Die Abtrennung von Butadien aus Roh-C₄-Schnitt erfolgt durch Extraktivdestillation unter Verwendung von N-Methylpyrrolidon.

Der Reaktionsaustrag der Extraktivdestillation wird gemäß einer Ausführungsform der Erfindung in den zweiten Schritt der vorangehend beschriebenen Selektivhydrierung eingespeist, um Restmengen Butadien zu entfernen und das gewünschte Molverhältnis von 1-Buten zu 2-Buten einzustellen.

### Abtrennung von i-Buten via Veretherung mit Alkoholen (Schritt C)

In der Veretherungsstufe wird i-Buten mit Alkoholen, vorzugsweise mit i-Butanol, an einem sauren Katalysator, vorzugsweise an einem sauren Ionenaustauscher, zu Ether, vorzugsweise i-Butyl-tert-butylether umgesetzt. Die Umsetzung erfolgt gemäß einer Ausführungsform der Erfindung in einer dreistufigen Reaktorkaskade, in der geflutete Festbettkatalysatoren von oben nach unten durchströmt werden. Im ersten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Bei einem Verhältnis von i-Butanol zu i-Buten von 0,8 bis 2,0, vorzugsweise 1,0 bis 1,5 beträgt der Umsatz zwischen 70 und 90%.
Im zweiten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Der Gesamtumsatz über die zwei Stufen erhöht sich auf 85 bis 99%, vorzugsweise 90 bis 97%.
Im dritten und größten Reaktor wird bei gleicher Eingangs- und Ausgangstemperatur von 0 bis 60°C, vorzugsweise 10 bis 50°C, der Gleichgewichtsumsatz erzielt.

An die Veretherung und Abtrennung des gebildeten Ethers schließt sich die Etherspaltung an, um Isobuten in hoher Reinheit zu gewinnen. Die endotherme Reaktion wird an sauren Katalysatoren, vorzugsweise an sauren Heterogenkontakten, beispielsweise Phosphorsäure auf einem SiO₂-Träger, bei einer Eingangstemperatur von 150 bis 300°C, vorzugsweise bei 200 bis 250°C, und einer Ausgangstemperatur von 100 bis 250°C, vorzugsweise bei 130 bis 220°C durchgeführt.

Bei Einsatz von FCC-C₄-Schnitt ist damit zu rechnen, daß Propan und i-Butan und andere Kohlenwasserstoffe in größeren Mengen eingeschleust werden, welche die nachfolgende Verfahrenssequenz beeinträchtigen können. Im Rahmen der Aufarbeitung des Ethers ist demzufolge die Möglichkeit einer destillativen Abtrennung der genannten Komponenten vorgesehen.

Der so erhaltene Reaktionsaustrag wird als Raffinat II bezeichnet und weist einen i-Buten-Restgehaltvon 0,1 bis 3 Gew.-% auf.

### Reinigung des Raffinat II-Stroms an Adsorbermaterialien (Schritt D)

Der nach der Veretherung (bzw. Destillation) erhaltene Raffinat II-Strom wird an mindestens einem Guard Bed aus hochoberflächigen Aluminiumoxiden, Kieselgelen, Alumosilikaten oder Molsieben gereinigt. Das Schutzbett dient hierbei zum Trocknen des C₄-Stroms sowie zur Entfernung von Substanzen, die als Katalysatorgift im nachfolgenden Metatheseschritt wirken können. Die Reinigung erfolgt in Trockentürmen vorzugsweise bei Temperaturen und Drücken, die so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenates, organischen Chloriden und Schwefelverbindungen. Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten, was bei der Abtrennung von i-Buten über IBTBE nicht notwendig ist.

### Zweistufige Metathesereaktion zur Herstellung von Propen (Schritt F)

Der so erhaltene Raffinat II-Strom, der 1-Buten, 2-Butene und Butane enthält, kann beispielsweise 70 bis 90 Gew.-% Olefine und 10 bis 30 Gew.-% Butane aufweisen, mit beispielsweise 25 bis 50 Gew.-% 1-Buten, 30 bis 55 Gew.-% 2-Buten, maximal 1 bis 2 Gew.-% Isobuten. Gemäß einer Ausführungsform der Erfindung kann der C₄-Strom geringe Mengen anderer Kohlenwasserstoffe enthalten.

Neben der Umsetzung von 1-Buten und 2-Buten zu Propen und 2-Penten kann in der Metathese ein geringer Anteil an 3-Hexen und Ethen als Nebenprodukt erhalten werden. Zudem können auch geringe Mengen höhersiedender Verbindungen vorliegen.

Die geringen Mengen an Nebenprodukten, die gemäß einer Ausführungsform der Erfindung 1 bis 30 Gew.-%, vorzugsweise 5 bis 20 Gew.-%, bezogen auf die Mengen an gebildetem 2-Penten, ausmachen, stören bei der nachfolgenden Umsetzung nicht. Gemäß einer Ausführungsform der Erfindung können 2-Penten und 3-Hexen als Produkte gewonnen werden.

Somit werden unter dem Ausdruck "2-Penten" auch solche Gemische verstanden, die neben 2-Penten geringe Mengen an Hexenen, insbesondere 3-Hexen, und anderen höhersiedenden Verbindungen enthalten. Entsprechend wird unter dem Ausdruck "Butene", wie "1-Buten" und "2-Buten" auch ein Gemisch verstanden, das neben dem Buten oder den Butenen C₄-Alkane, insbesondere Butane enthält.

Im ersten Metatheseschritt wird Raffinat II an einem heterogenen Disproportionierungskatalysator, vorzugsweise einem Rheniumoxid auf Aluminiumoxid, umgesetzt. Der Katalysator wird dabei durch Tränken des Trägers mit wäßriger Ammoniumperrhenat- oder Perrheniumsäure-Lösung oder durch Aufsprühen der Lösungen, anschließendem Trocknen und Calzinieren erhalten. Die Reaktion erfolgt bei Temperaturen zwischen 0°C und 100°C, vorzugsweise bei 20 bis 90°C sowie einem Druck, der mindestens geringfügig höher ist als der Dampfdruck der eingesetzten C₄-Komponenten. Die Umsetzung wird in Rohrreaktoren bzw. Schachtöfen im Swingmodus Synthese/Regeneration durchgeführt.

In diesem ersten Metatheseschritt entstehen aus 1-Buten und 2-Buten in der Hauptreaktion Propen und 2-Penten sowie in einer Nebenreaktion Ethen und 3-Hexen. Die Durchführung der Reaktion erfolgt in der Art, daß das Austragsverhältnis Propen zu 2-Penten maximiert wird. Der Gesamt-C₄-Umsatz liegt in diesem Schritt bei 30 bis 70%, vorzugsweise bei 40 bis 65%.

Der Reaktionsaustrag durchläuft nachfolgend einen Destillationsschritt: Die hierzu eingesetzte Druckkolonne kann als Trennwandkolonne, Seitenkolonne, Seitenabzugskolonne oder Zweikolonnenschaltung ausgelegt sein und dient zur Trennung des Reaktionsaustrags in eine C₂/C₃-Leichtsiederphase, eine C₄-Mittelsiederphase und eine C₅/C₆-Hochsiederphase. Druck und Temperaturverläufe in der Kolonne werden entsprechend der genannten Trennaufgabe eingestellt. Gegebenenfalls gebildete höhersiedende Komponenten können im Kolonnensumpf ausgeschleust werden.

Im zweiten Metatheseschritt wird der in der vorangehend beschriebenen Destillation erhaltene C₅/C₆-Hochsiederaustrag zumindest teilweise zusammen mit Ethen an einem heterogenen Disproportionierungskatalysator, vorzugsweise einem Rheniumoxid auf Aluminiumoxid, umgesetzt. Der Katalysator wird dabei durch Tränken des Trägers mit wäßriger Ammoniumperrhenat- oder Perrheniumsäure-Lösung oder durch Aufsprühen der Lösungen, anschließendem Trocknen und Calzinieren erhalten. Die Reaktion erfolgt bei Temperaturen zwischen 0°C und 100°C, vorzugsweise bei 20 bis 90°C sowie einem Druck, der mindestens geringfügig höher ist als der Dampfdruck der eingesetzten Komponenten. Vorzugsweise liegt der Druck bei 30 bis 70 bar. Auch diese Umsetzung wird in Rohrreaktoren bzw. Schachtöfen im Swingmodus Synthese/Regeneration durchgeführt.

In diesem zweiten Metatheseschritt entstehen aus dem die Olefine 2-Penten und 3-Hexen enthaltenden C₅/C₆-Hochsiederaustrag bei Einspeisung von Ethen, wobei das Molverhältnis C₂- C₅/C₆ zwischen 0,5 und 5, vorzugsweise zwischen 0,8 und 2,0 liegt, Propen und 1-Buten. Der Gesamt-Umsatz liegt in diesem Schritt bei 30 bis 95%, vorzugsweise bei 50 bis 80%. Der Reaktionsaustrag des zweiten Metatheseschrittes wird ebenfalls in die vorangehend beschriebene Destillationskolonne zur Trennung des Reaktionsaustrags in eine C₂/C₃-Leichtsiederphase, eine C₄-Mittelsiederphase und eine C₅/C₆-Hochsiederphase eingespeist. Gegebenenfalls gebildete höhersiedende Komponenten können im Kolonnensumpf ausgeschleust werden.

Der C₂/C₃-Leichtsiederaustrag der Kolonnen wird einer weiteren Destillationskolonne zugeführt, in welcher die Propen-Feindestillation erfolgt. Abgetrenntes Ethen wird zumindest teilweise in die zweite Metathesestufe zurückgeführt.

Der C₄-Mittelsiederaustrag der Kolonne wird zumindest teilweise in die erste Metathesestufe rückgeführt. Frisch-C₄-Feed und C₄-Rückführstrom werden so kombiniert, daß in den ersten Metatheseschritt ein Feedstrom gelangt, welcher ein 1-Buten zu 2-Buten-Molverhältnis von 5 zu 1 bis 1 zu 5 aufweist.

Mehrere Ausführungsformen von Schritt E der Erfindung werden nachstehend anhand der Zeichnung erläutert, in der
- Figur 1: eine schematische Darstellung einer Ausführungsform des erfindungsgemäßen Verfahrensschrittes E und
- Figur 2: eine schematische Darstellung einer weiteren Ausführungsform des erfindungsgemäßen Verfahrensschrittes E zeigen.

Dabei bedeuten die in den Figuren verwendeten Abkürzungen folgendes:
- 1-Bu:: 1-Buten
- 2-Bu:: 2-Buten
- Bu:: Butane
- Et:: Ethen
- Pr:: Propen
- 2-Pe:: 2-Penten
- 3-He:: 3-Hexen
- H:: Hochsieder
- II:: Raffinat II
- C4:: C4-Olefine
- C5⁺:: Olefine mit 5 oder mehr Kohlenstoffatomen
- R01:: Reaktor (Metathese)
- R02:: Reaktor (Metathese)
- R03:: Reaktor (Isomerisierung)
- K101:: Destillationskolonne (vorzugsweise eine Trennwandkolonne, Seitenkolonne oder 2-Kolonnen-Schaltung)
- K201:: Destillationskolonne (vorzugsweise eine Trennwandkolonne, Seitenkolonne oder 2-Kolonnen-Schaltung)
- K301:: Destillationskolonne

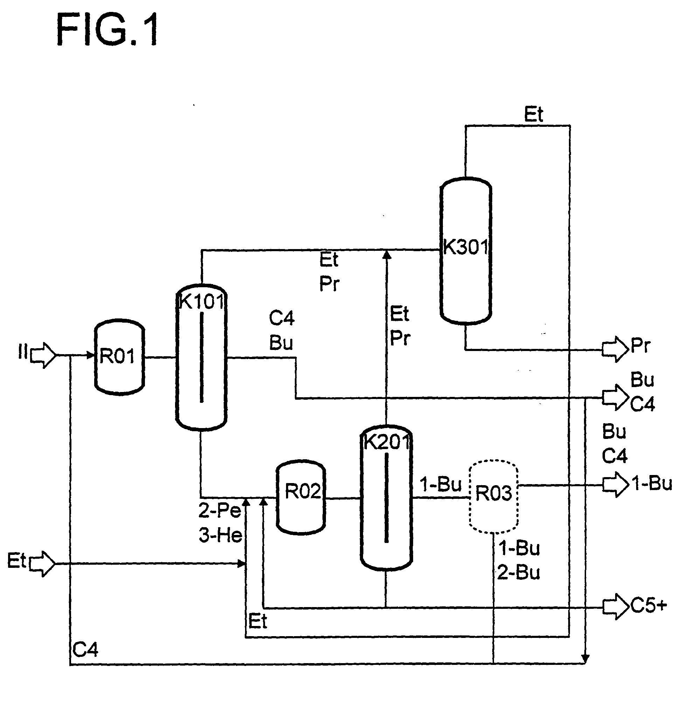

Nachstehend wird eine bevorzugte Ausführungsform des erfindungsgemäßen Verfahrensschrittes E beschrieben, die in Fig. 1 dargestellt ist.

In einem ersten Reaktor R01 werden in Gegenwart des erfindungsgemäßen Metathesekatalysators 1-Buten und 2-Buten zu Propen und 2-Penten umgesetzt. Dazu wird ein Raffinat II-Strom in den Reaktor geführt. An den Reaktor schließt sich eine als Trennwandkolonne, Seitenkolonne oder 2-Kolonnen-Schaltung ausgelegte Destillationskolonne K101 an, an deren Kopf Propen und als Nebenprodukt gebildetes Ethen entfernt werden. Nicht umgesetztes Raffinat II wird im Mittelabzug entfernt und teilweise in den Feedstrom von Raffinat II zurückgeführt. Es kann auch teilweise ausgeschleust werden. Aus dem Sumpf von K101 werden 2-Penten und als Nebenprodukt gebildetes 3-Hexen sowie Hochsieder entnommen. Der Sumpf wird sodann zusammen mit eingespeistem Ethen einem Reaktor R02 zugeführt, der wiederum einen erfindungsgemäßen Metathesekatalysator enthält. In diesem Reaktor R02 findet die Umsetzung von 2-Penten mit Ethen zu 1-Buten und Propen statt. Der Austrag aus Reaktor R02 wird einer als Trennwandkolonne, Seitenkolonne oder 2-Kolonnen-Schaltung ausgelegten Destillationskolonne K201 zugeführt, an deren Kopf Propen und nicht umgesetztes Ethen abgeführt werden. Im Mittelabzug wird gebildetes 1-Buten gegebenenfalls teilweise ausgetragen, und gegebenenfalls zumindest teilweise in den Isomerisierungsreaktor R03 geführt. Im Sumpf von K201 fallen nicht umgesetztes 2-Penten, sowie als Nebenprodukte 3-Hexen und Hochsieder an. Diese werden ausgeschleust oder vorzugsweise in R02 zurückgeführt. Die aus K101 und K201 am Kopf abgeführten Gemische aus Propen und Nebenprodukt Ethen werden in einer weiteren Destillationskolonne K301 aufgetrennt. Am Kopf von K301 wird Ethen gewonnen, das vorzugsweise in den Reaktor R02 (zurück)geführt wird. Im Isomerisierungsreaktor R03 wird das 1-Buten an einem Isomerisierungskatalysator zumindest teilweise zu 2-Buten isomerisiert, und das Isomerisierungsgemisch wird in den Reaktor R01 zurückgeführt. Die unterbrochene Linie in R03 deutet die mögliche Ausschleusung des 1-Buten an. Das im Sumpf von K301 erhaltene Propen ist -neben gegebenenfalls ausgeschleusten 1-Buten aus K201 - das gewünschte Umsetzungsprodukt des erfindungsgemäßen Verfahrens. K101 und K201 sind so ausgelegt, daß am Kopf der Kolonne eine leichtsiedende Phase, insbesondere eine C_{2/3}-Phase, die Ethen und Propen enthält, abgeführt wird. Als Mittelsiederphase werden C₄-Ströme abgeführt, insbesondere Butene und Butane. In der Sumpfphase werden C_{≥5}-Kohlenwasserstoffe ausgetragen.

Zwischen den Schritten b) und c) kann der abgetrennte, 2-Penten und 3-Hexen enthaltende Hochsiederaustrag einer Destillation zur Trennung von 2-Penten und 3-Hexen unterworfen werden. Die Destillation kann in jeder geeigneten Apparatur durchgeführt werden. Die 2-Penten enthaltende Fraktion wird danach dem Reaktor R02 zugeführt. Das 2-Penten und/oder 3-Hexen kann ausgeschleust und beispielsweise einer Dimerisierung zu einem C₁₀ - bzw. C₁₂ -Olefingemisch zugeführt werden.

Die Reaktoren R01, R02 und R03 können beliebige zur kontinuierlichen Reaktionsführung geeignete Reaktoren sein. Gemäß einer Ausführungsform können die Reaktoren Rohrreaktoren oder Reaktionskolonnen sein. Bevorzugt sind Rohrreaktoren.

Bei den Destillationskolonnen K101 und K201 handelt es sich gemäß einer Ausführungsform der Erfindung um solche Kolonnen, die eine Auftrennung eines Kohlenwasserstoffstroms in C_{2/3}-Ströme, C₄-Ströme und C_{≥5}-Ströme erlauben. Die Kolonnen können als Trennwandkolonnen, Seitenkolonnen oder als 2-Kolonnen-Schaltungen ausgeführt sein. K301 ist gemäß einer Ausführungsform der Erfindung eine Kolonne, die die Trennung von Ethen und Propen erlaubt. Gemäß einer Ausführungsform der Erfindung ist der Reaktor R01 mit der Destillationskolonne K101 kombiniert zu einer Reaktivdestillationseinrichtung. Dabei erfolgt die Umsetzung direkt in der Destillationskolonne. Der Katalysator liegt in der Reaktionskolonne vor, so daß gleichzeitig mit der Umsetzung oder unmittelbar darauffolgend die Destillation ausgeführt wird. Ein entsprechendes Verfahren ist unter der Bezeichnung "Reaktivdestillation" bekannt.

Gemäß einer Ausführungsform sind Reaktor R02 und Destillationskolonne K201 zusammengefaßt zu einer Reaktivdestillationsvorrichtung, bei der Umsetzung und Destillation kombiniert sind entsprechend der vorstehend beschriebenen Reaktivdestillation.

Gemäß einer Ausführungsform der Erfindung finden beide Umsetzungsreaktionen in Reaktivdestillationsvorrichtungen statt. Bei beiden Umsetzungen handelt es sich um Gleichgewichtsreaktionen, so daß gemäß einer Ausführungsform der Erfindung die Verfahrensprodukte zur Erzielung eines möglichst hohen Umsatzes möglichst schnell aus dem Gleichgewicht entfernt werden. Dies ist insbesondere bei Verwendung von Reaktivdestillationsvorrichtungen möglich.

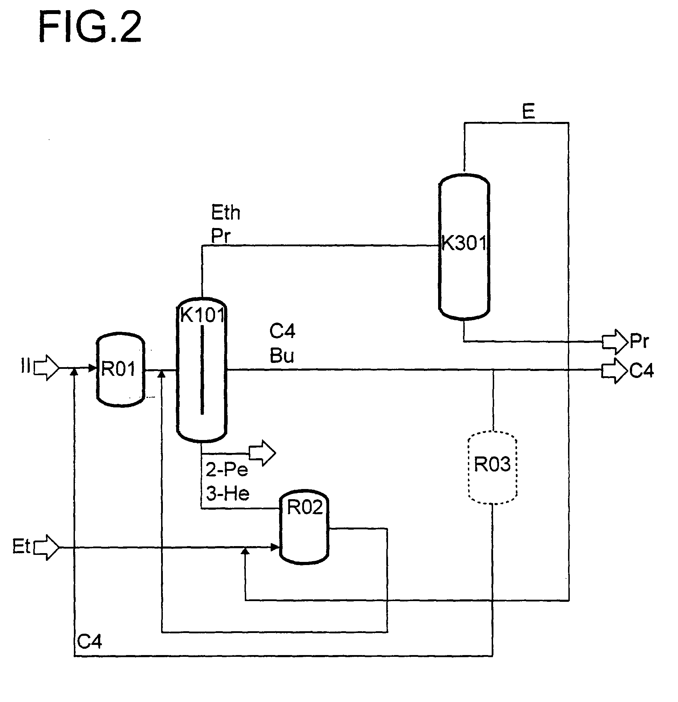

Eine weitere Ausführungsform des erfindungsgemäßen Verfahrensschrittes F ist in Figur 2 dargestellt.

Das aus dem zweiten Metathesereaktor R02 erhaltene Gemisch wird direkt in die Destillationskolonne K101 zurückgeführt. Der in der Kolonne K101 erhaltene Mittelsiederaustrag aus C₄-Olefinen und Butanen wird gegebenenfalls zumindest teilweise ausgeschleust und/oder gegebenenfalls zumindest teilweise im Isomerisierungsreaktor R03 umgesetzt, wobei 1-Buten zu 2-Buten isomerisiert wird. Der Austrag aus dem Isomerisierungsreaktor R03 wird in Schritt a), d.h. den Metatheseraktor R01, zurückgeführt. Bei dieser Verfahrensvariante kann die Destillationskolonne K201 eingespart werden.

### METATHESE-KATALYSATOR

In den erfindungsgemäßen Verfahren können in R01 und R02 alle geeigneten Metathesekatalysatoren eingesetzt werden.

Gemäß einer Ausführungsform der Erfindung ist der Katalysator ein heterogener Katalysator, insbesondere ein Trägerkatalysator. Gemäß einer Ausführungsform der Erfindung enthält der Katalysator mindestens eine Verbindung eines Metalls der VIb, VIIb oder VIII Nebengruppe des Periodensystems der Elemente. Vorzugsweise enthält der Katalysator eine Rheniumverbindung. Derartige Katalysatoren sind beispielsweise beschrieben in K.J. Ivin, I.C. Mol, Olefin Metathesis and Metathesis Polymerization, 2nd Edition, Academic Press, New York, 1996; G.W. Parshall, S.D. Ittel, Homogeneous Catalysis, 2nd Edition, 1992, John Wiley & Sons, New York, Chichester, Brisbane, Toronto, Singapur, S. 217ff; R.H. Grubbs in Prog. Inorg. Chem., S. Lippard (Hrsg.), John Wiley & Sons, New York, 1978, Vol. 24, 1-50; R.H. Grubbs in Comprehensive Organomet. Chemie., G. Wilkinson (Hrsg.), Pergamon Press, Ltd., New York, 1982, Vol. 8, 499-551; D.S. Breslow, Prog. Polym. Sci. 1993, Vol. 18, 1141-1195.

Gemäß einer Ausführungsform der Erfindung handelt es sich bei der Metallverbindung um ein Metalloxid, Teiloxid mit zusätzlich vorliegenden organischen Resten oder um eine Carbonylverbindung.

Vorzugsweise ist die Metallverbindung ein Oxid von Rhenium, insbesondere Re₂O₇.

### TRÄGER

Die erfindungsgemäßen Katalysatoren enthalten gemäß einer Ausführungsform der Erfindung einen Träger. Als Träger kommen dabei insbesondere anorganische Träger zur Anwendung, wie Al₂O₃, insbesondere γ-Al₂O₃, SiO₂, Fe₂O₃, oder deren Gemische, wie SiO₂/Al₂O₃, B₂O₃/SiO₂/Al₂O₃ oder Fe₂O₃/Al₂O₃.

Der Metalloxidgehalt auf dem Träger beträgt dabei gemäß einer Ausführungsform der Erfindung 1 bis 20 Gew.-%, vorzugsweise 3 bis 15 Gew.-%, insbesondere 8 bis 12 Gew.-%, bezogen auf das Gesamtgewicht des Trägerkatalysators.

Bevorzugt wird als Katalysator Re₂O₇ auf Al₂O₃, SiO₂/Al₂O₃, SiO₂/Al₂O₃/Fe₂O₃ oder B₂O₃/Al₂O₃ verwendet. Dabei beträgt der Anteil an Metalloxid vorzugsweise 1 bis 20 Gew.-%, besonders bevorzugt 3 bis 10 Gew.-%. Gemäß einer Ausführungsform der Erfindung wird MeReO₃ anstelle von Re₂O₇ oder im Gemisch damit verwendet.

Besonders bevorzugt wird erfindungsgemäß Re₂O₇ auf Al₂O₃ verwendet.

Die Katalysatoren werden gemäß einer Ausführungsform der Erfindung frisch calciniert eingesetzt, wobei sie keiner weiteren Aktivierung, beispielsweise durch Alkylierungsmittel bedürfen. Desaktivierte Katalysatoren können erfindungsgemäß durch Abbrennen von Coke-Rückständen beispielsweise bei 550°C im Luftstrom und Abkühlung unter Inertgas regeneriert werden.

Die erfindungsgemäßen Umsetzungen können dabei in Gegenwart eines Lösungsmittels, beispielsweise eines Kohlenwasserstofflösungsmittels durchgeführt werden. Gemäß einer bevorzugten Ausführungsform der Erfindung werden die Umsetzungen ohne weiteres zugesetztes Lösungsmittel durchgeführt.

### ISOMERISIERUNGS-KATALYSATOR

Als Isomerisierungs-Katalysator können alle Katalysatoren eingesetzt werden, die die Isomerisierung von 1-Buten zu 2-Buten katalysieren. Geeignete Isomerisierungskatalysatoren sind prinzipiell sämtliche homogenen oder heterogenen Edelmetallverbindungen, gegebenenfalls in Gegenwart von Wasserstoff, wie beispielsweise in A.J. Hubert, H. Reimbinger, Synthesis 1970,1,405 beschrieben.

Beispielsweise kann die Isomerisierung gemäß US 3,592,868 an RuO₃ auf einem anorganischen Träger wie SiO₂, Al₂O₃ oder TiO₂ oder gemischten Trägern erfolgen. In US 4,684,760 und US 4,889,840 sind Mischkatalysatoren, bestehend aus Magnesiumoxid, Zirkoniumoxid und einem Alkalimetalloxid auf einem anorganischen Träger beschrieben. In EP-A-0 129 899 und DE-A-34 27 979 sind geeignete Phosphorsäuren und Phosphate enthaltende Verbindungen sowie Zeolithe des Pentasil-Typs beziehungsweise übergangsmetall-dotierte Zeolithe beschrieben. Vorteilhaft hinsichtlich Katalysator- Standzeit und Reaktionsbedingungen sind die in US 5,177,281 beschriebenen Zeolithe des ZSM-Typs wie ZSM-22, ZSM-23 oder ZSM-35. Besonders aktive Palladium-Katalysatoren, beispielsweise auf Al₂O₃ als Träger, sind in US 3,531,545 beschrieben.

Der Isomerisierungskatalysator ist vorzugsweise ein heterogener Katalysator, der eine Verbindung eines Edelmetalls aus der Nebengruppe des Periodensystems der Elemente enthält, das in Form des Metalls oder eines Oxids oder Mischoxids vorliegen kann. Des weiteren sind Verbindungen eines Metalls der I. oder II. Hauptgruppe des Periodensystems der Elemente geeignet, die als Oxid oder Mischoxid vorliegen können.

Vorzugsweise wird ein Metall oder Metalloxid aus der VII. und VIII. Nebengruppe des Periodensystems der Elemente, das auf einem Träger vorliegen kann, in Inertgasatmosphäre oder in Gegenwart von Wasserstoff als Isomerisierungskatalysator eingesetzt.

Die bevorzugten erfindungsgemäßen Alkali- und/oder Erdalkalimetalloxidkatalysatoren werden vorzugsweise durch Tränkung von anorganischen Trägern wie SiO₂, Al₂O₃, ZrO₂, TiO₂ oder Gemischen davon mit Alkali- und/oder Erdalkaliverbindungen, nachfolgende Trocknung und Calzinieren zu den entsprechenden Oxiden hergestellt. Desaktivierter Katalysator kann durch Abbrennen von Coke-Rückständen bei Temperaturen oberhalb 350°C im Luftstrom und Abkühlung unter Inertgas-Atmosphere einfach regeneriert werden.

Besonders bevorzugt wird als Isomerisierungskatalysator PdO auf einem Al₂O₃- oder SiO₂-Träger in Gegenwart von Wasserstoff eingesetzt, wobei der Gehalt an Pd 0,1 bis 5 Gew.-%, bezogen auf das Gesamtgewicht des Katalysators, beträgt.

Beim Einsatz der vorstehenden Katalysatoren wird die C₄-Mittelsiederfraktion aus der Kolonne K201 nach partieller Isomerisierung in ein Gemisch aus 1-Buten und 2-Butenen überführt und zur Erhöhung der Propenausbeute in die Kreuzmetathese in R01 zurückgeführt. Alternativ kann 1-Buten bei entsprechender Reinheit des Raffinat II-Feedstroms ohne weitere Aufarbeitung gewonnen werden. Es kann dann beispielsweise zur Herstellung von Polymeren wie LLDPE-Copolymeren, HDPE-Copolymeren, Poly-1-buten oder zur Herstellung von Butylenoxid eingesetzt werden.

Bei der Isomerisierung werden - ebenso wie bei den Metathesereaktionen in R01 und R02 - die Bedingungen so gewählt, daß die Reaktionspartner in flüssiger Phase vorliegen. Die Temperatur beträgt so vorzugsweise 0 bis 200°C, besonders bevorzugt 50 bis 150°C. Der Druck beträgt vorzugsweise 2 bis 200 bar. Die Isomerisierung ist vorzugsweise nach einer Sekunde bis eine Stunde, vorzugsweise 5 bis 30 Minuten beendet. Sie kann kontinuierlich oder diskontinuierlich durchgeführt werden, wobei die Reaktoren wie die Metathesereaktoren Druckglas-Gefäße, Rohrreaktoren oder Destillationskolonnen sein können. Vorzugsweise werden auch hier Rohrreaktoren eingesetzt.

Die Erfindung wird nachstehend anhand von Beispielen näher erläutert.

### BEISPIELE

### Beispiel 1

### Kontinuierlicher Versuch zur zweistufigen Selektivhydrierung von Roh-C₄-Schnitt

Roh-C₄-Schnitt mit einer Zusammensetzung von 43,7% Butadien (einschließlich Butenin und Butin), 14,3% 1-Buten, 7,8% 2-Butene und 7,2% n-Butan wurde in einem kontinuierlich durchströmten Rohrreaktor an 0,3% Pd/Al₂O₃-Heterogenkontakt bei einem Frischzulauf von 1kg/h Roh-C₄ und einem Kreislauf von 8,2 kg/h mit einer LHSV von 9,0 h⁻¹ bei einer Reaktoreingangstemperatur von 20°C mit 175 Nl/h Wasserstoff umgesetzt. Bei einem Butadien-Umsatz von 95,2% wurde in der ersten Stufe der Selektivhydrierung unter diesen Bedingungen eine Gesamt-Buten-Selektivität von 99,6 % sowie eine 1-Buten-Selektivität von 56,5% erzielt.

Ein typischer Reaktionsaustrag aus der ersten Stufe der Selektivhydrierung aus 0,61% Butadien (einschließlich Butenin und Butin), 26,9% 1-Buten, 14,9% 2-Butene und 11,6% n-Butan wurde in einem kontinuierlich durchströmten Rohrreaktor an 0,3 % Pd/Al₂O₃-Heterogenkontakt bei einem Frischzulauf von 2,2 kg/h Reaktionsaustrag der 1. Stufe und einem Kreislauf von 4,0 kg/h mit einer LHSV von 20 h⁻¹ bei einer Reaktoreingangstemperatur von 60°C und einer Reaktorausgangstemperatur von 70°C mit 16 Nl/h Wasserstoff umgesetzt. Bei einem Butadien-Umsatz von 99,2% wurde unter diesen Bedingungen bei einem 1-Buten-Erhalt von 58,2% ein Raffinat I-Strom erhalten, der einen Restgehalt von 48 ppm Butadien aufwies.

### Beispiel 2

### Kontinuierlicher Versuch zur Abtrennung von i-Buten durch Veretherung mit i-Butanol

In einer dreistufigen Reaktorkaskade wurde ein geflutetes und mit saurem Ionenaustauscher bestücktes Festbett von oben nach unten mit Raffinat I und i-Butanol durchströmt, wobei das Verhältnis von i-Butanol zu i-Buten im Feed auf 1,2 eingestellt wurde. Die Reaktoreingangstemperatur lag bei 40°C, die Reaktorausgangstemperatur bei 65°C und der Reaktionsdruck bei 8 bar. Der gemessene i-buten-Umsatz nach der ersten Stufe lag bei 85%.

Im zweiten, ähnlich dimensionierten Reaktor wurde der Umsatz bei einer Reaktoreingangstemperatur bei 40°C, einer Reaktorausgangstemperatur bei 50°C und einem Reaktionsdruck von 8 bar auf 95% erhöht.

Im dritten, deutlich größeren Reaktor wurde der Gleichgewichtsumsatz bei einer Reaktoreingangstemperatur und Reaktorausgangstemperaturvon jeweils 40°C und einem Reaktionsdruck von 8 bar eingestellt. Der unter diesen Bedingungen nach destillativer Abtrennung von i-Butyl-tert-butylether verbleibende Raffinat-Strom wies einen i-Buten-Restgehalt von 0,7% auf, der den nachfolgenden Metatheseschritt nicht beeinträchtigte.

### Beispiel 3

### Kontinuierlicher Versuch zur zweistufigen Metathese von Raffinat II

Ein nach Beispiel 2 erhaltener Raffinat-Strom (1,05 kg/h, 85% n-Buten-Anteil) wurde mit C₄-Rückführstrom (2,82 kg, 55% n-Buten-Anteil) vermischt und nach Feedreinigung über Molsieb 13X bei 40°C und 10 bar kontinuierlich über einen mit Re₂O₇/Al₂O₃-Heterogenkontakt bestückten Rohrreaktor geleitet. Der Reaktionsaustrag gelangte in eine Druckdestillation (20 bar), in welcher eine C_{2/3}-Leichtsiederphase aus 83% Propen und 17% Ethen, welche nachfolgend feindestilliert wurden, eine C₄-Olefine und Butane enthaltende Mittelsiederfraktion, von der 10% ausgeschleust wurde, sowie eine aus 2-Penten und 3-Hexen bestehende Hochsiederfraktion getrennt wurden. Letztere wurde nach Teilausschleusung von C₅-Kohlenwasserstoffen in der Ethenolysereaktion, die bei 40°C, 35 bar Ethen kontinuierlich an einem Re₂O₇/Al₂O₃-Heterogenkontaktbestückten Rohrreaktor durchgeführt wurde, mit 0,5 kg/h Ethen zu einem aus Propen und 1-Buten bestehenden Ethenolyse-Austragsstrom mit 22 Gew.-% Propen umgesetzt. Der Ethenolyse-Austragsstrom wurde ebenfalls in die Druckdestillation eingespeist.

## Patentansprüche

1. Verfahren zur Herstellung von Olefinen aus Steamcracker- oder Raffinerie-C₄-Strömen durch
A) gegebenenfalls Extraktivdestillation von Butadien aus dem C₄-Strom mit einem Butadien-selektiven Lösungsmittel, um einen weitgehend Butadien-abgereicherten C₄-Strom zur erhalten,
B) Selektivhydrierung von Butadienen und acetylenischen Verunreinigungen im Steamcracker- oder Raffinerie-C₄-Strom oder dem Strom aus Schritt A), mit gleichzeitiger oder nachgeschalteter zumindest teilweiser Isomerisierung von 1-Buten zu 2-Buten, um einen C₄-Strom zu erhalten, der n-Butene und i-Buten enthält und im wesentlichen frei von Butadienen und acetylenischen Verunreinigungen ist,
C) Entfernung von i-Buten aus dem in Schritt B) erhaltenen C₄-Strom durch Umsetzung mit einem Alkohol zur Bildung eines Ethers, der abgetrennt und gegebenenfalls zur Gewinnung von reinem Isobuten rückgespalten wird, um einen C₄-Strom zu erhalten, der n-Butene und gegebenenfalls Oxygenat-Verunreinigungen enthält, und aus dem bei der Abtrennung des Ethers auch niedriger und höher siedende Verbindungen destillativ entfernt werden können,
D) Abtrennen von Oxygenat-Verunreinigungen aus dem in Schritt C) erhaltenen C₄-Strom mit Adsorbermaterialien,
E) zweistufige Metathese der Butene im in Schritt D) erhaltenen C₄-Strom durch
a) Umsetzung von im C₄-Strom enthaltenem 1-Buten und 2-Buten zu Propen und 2-Penten in Gegenwart eines Metathesekatalysators, der mindestens eine Verbindung eines Metalls der VIb, VIIb oder VIII Nebengruppe des Periodensystems der Elemente enthält,
b) anschließendes Trennen des gebildeten Propens und 2-Pentens und der nicht umgesetzten Butene, und gegebenenfalls zumindest teilweises Ausschleusen einer oder mehrerer dieser Verbindungen,
c) anschließende Umsetzung des 2-Pentens mit Ethen zu Propen und 1-Buten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens,
e) gegebenfalls zumindest teilweises Ausschleusen eines reinen 1-Buten-Stroms und/oder gegebenenfalls zumindest teilweises Isomerisieren des gebildeten 1-Butens zu 2-Buten in Gegenwart eines Isomerisierungskatalysators und anschließendes Rückführen des nicht ausgeschleusten 1-Butens und gegebenfalls gebildeten 2-Butens gemeinsam mit einem Teil der in Schritt a) nicht umgesetzten C₄-Fraktion in Schritt a),
f) Gewinnen des in den Schritten b) und d) abgetrennten Propens,
wobei die Trennung in Schritt d) durch Überführen des umgesetzten Gemisches in Schritt b) erfolgen kann, wobei dann in Schritt e) die in Schritt b) abgetrennte, nicht umgesetzte C₄-Fraktion gegebenenfalls zumindest teilweise ausgeschleust und/oder gegebenenfalls das in dieser C₄-Fraktion enthaltene 1-Buten in Gegenwart eines Isomerisierungskatalysators zumindest teilweise zu 2-Buten isomerisiert wird und das erhaltene Gemisch anschließend in Schritt a) zurückgeführt wird.

2. Verfahren nach Anspruch 1, wobei als Olefin Propen gewonnen wird.

3. Verfahren nach Anspruch 1 oder 2, wobei Schritt E b) eine Destillation ist, die in einer Trennwandkolonne, Seitenkolonne oder 2-Kolonnen-Schaltung durchgeführt werden kann, in der eine Propen enthaltende Leichtsiederphase, gegebenenfalls eine Butene enthaltende Mittelsiederphase und eine 2-Penten enthaltende Sumpfphase erhalten werden,
und/oder
wobei Schritt E d) eine Destillation ist, die in einer Trennwandkolonne durchgerührt werden kann, in der eine Propen enthaltende Leichtsiederphase, eine 1-Buten enthaltende Mittelsiederphase und gegebenenfalls eine 2-Penten enthaltende Sumpfphase erhalten werden, wobei Schritte E b) und E d) in einer Destillationskolonne durchgeführt werden können.

4. Verfahren nach einem der Ansprüche 1 bis 3, wobei die Umsetzung in Schritten E a) und/oder E c) nicht vollständig erfolgt und in Schritt E b) und/oder E d) eine C_{2/3} enthaltende Leichtsiederphase, eine C₄-Mittelsiederphase und eine C_{≥5} enthaltende Sumpfphase erhalten werden,
wobei die gegebenenfalls zusammengefaßten Leichtsiederphasen durch Destillation in C₂- und C₃-Phasen getrennt werden und die C₂-Phase in Schritt E c) zurückgeführt wird,
die gegebenenfalls zusammengefaßten Mittelsiederphasen zumindest teilweise in Schritt E a) zurückgeführt werden und die gegebenenfalls zusammengefaßten Sumpfphasen zumindest teilweise in Schritt c) zurückgeführt werden.

5. Verfahren nach einem der Ansprüche 1 bis 4, **dadurch gekennzeichnet, daß** Schritt B bei Durchführung von Schritt A) einstufig und ohne Durchführung von Schritt A) zweistufig durchgeführt wird bei gleichzeitiger Isomerisierung von 1-Buten zu 2-Buten durch Inkontaktbringen des C₄-Stroms in flüssiger Phase mit einem Katalysator, der mindestens ein Metall, ausgewählt aus der Gruppe Nickel, Palladium und Platin auf einem Träger enthält, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen C₄-Strom zu erhalten.

6. Verfahren nach einem der Ansprüche 1 bis 5, **dadurch gekennzeichnet, daß** in Schritt A) das Butadien-selektive Lösungsmittel ausgewählt ist aus der Klasse polar-aprotischer Lösungsmittel, vorzugsweise aus Aceton, Furfurol, Acetonitril, Dimethylacetamid, Dimethylformamid und N-Methylpyrrolidon.

7. Verfahren nach einem der Ansprüche 1 bis 6, **dadurch gekennzeichnet, daß** die Veretherung von i-Buten in Schritt C) in einer Reaktorkaskade mit Methanol oder i-Butanol, vorzugsweise i-Butanol in Gegenwart eines sauren Katalysators, vorzugsweise eines saueren Ionenaustauschers durchgeführt wird, in der geflutete Festbettkatalysatoren von oben nach unten durchströmt werden, wobei die Reaktor-Eingangstemperatur 0 bis 60°C, vorzugsweise 10 bis 50°C, die Ausgangstemperatur 25 bis 85, vorzugsweise 35 bis 75°C, der Druck 2 bis 50 bar, vorzugsweise 3 bis 20 bar, und das Verhältnis von i-Butanol zu i-Buten 0,8 bis 2,0, vorzugsweise 1,0 bis 1,5 beträgt und der Gesamtumsatz dem Gleichgewichtsumsatz entspricht.

8. Verfahren nach einem der Ansprüche 1 bis 7, **dadurch gekennzeichnet, daß** Schritt D) an mindestens einem Guard Bed aus hochoberflächigen Aluminiumoxiden, Kieselgelen, Alumosilikaten und/oder Molsieben durchgeführt wird, wobei der Reinigungsschritt zur Feed-Vorwärmung für den nachfolgenden Metatheseschritt eingesetzt werden kann.

9. Verfahren nach einem der Ansprüche 1 bis 8, wobei in Schritt E) ein heterogener Metathesekatalysator eingesetzt wird, der eine Rheniumverbindung enthält, vorzugsweise ein Rheniumoxid auf einem anorganischen Träger.

## Claims

1. A process for preparing olefins from steam cracker or refinery C₄ streams by
A) if desired, extractive distillation of butadiene from the C₄ stream using a butadiene-selective solvent to obtain a substantially butadiene-depleted C₄ stream,
B) selective hydrogenation of butadienes and acetylenic impurities in the steam cracker or refinery C₄ stream or the stream from step A) with simultaneous or subsequent, at least partial isomerization of 1-butene to 2-butene in order to obtain a C₄ stream which comprises n-butenes and i-butene and is essentially free of butadienes and acetylenic impurities,
C) removal of i-butene from the C₄ stream obtained in step B) by reaction with an alcohol to form an ether which is separated off and, if desired, redissociated to give pure isobutene, resulting in a C₄ stream which comprises n-butenes and possibly oxygen-containing impurities and from which lower- and higher-boiling compounds can also be removed by distillation during the removal of the ether,
D) separation of oxygen-containing impurities from the C₄ stream obtained in step C) using adsorber materials,
E) two-stage metathesis of the butenes in the C₄ stream obtained in step D) by
a) conversion of the 1-butene and 2-butene present in the C₄ stream into propene and 2-pentene in the presence of a metathesis catalyst comprising at least one compound of a metal of transition group VIb, VIIb or VIII of the Periodic Table of the Elements,
b) subsequent separation of the propene and 2-pentene formed and the unreacted butenes and, if desired, at least partial discharge of one or more of these compounds,
c) subsequent reaction of the 2-pentene with ethene to form propene and 1-butene in the presence of a metathesis catalyst comprising at least one compound of a metal of transition group VIb, VIIb or VIII of the Periodic Table of the Elements,
d) subsequent separation of the propene and 1-butene formed,
e) if desired, at least partial discharge of a pure 1-butene stream and/or, if desired, at least partial isomerization of the 1-butene formed to 2-butene in the presence of an isomerization catalyst and subsequent recirculation of the 1-butene which has not been discharged and the 2-butene which may have formed together with part of the C₄ fraction which has not been converted in step a) to step a),
f) isolation of the propene separated off in steps b) and d),
wherein the separation in step d) can be carried out by passing the reacted mixture to step b), wherein, subsequently in step e), the unreacted C₄ fraction separated off in step b) is, if desired, at least partly discharged and/or, if desired, the 1-butene present in this C₄ fraction is at least partially isomerized to 2-butene in the presence of an isomerization catalyst and the resulting mixture is subsequently recirculated to step a).

2. A process as claimed in claim 1, wherein the olefin isolated is propene.

3. A process as claimed in claim 1 or 2, wherein step E b) is a distillation which can be carried out in a dividing-wall column, side column or 2-column arrangement and in which a propene-containing low boiler phase, if desired a butene-containing intermediate boiler phase and a 2-pentene-containing bottom phase are obtained,
and/or
step E d) is a distillation which can be carried out in a dividing-wall column and in which a propene-containing low boiler phase, a 1-butene-containing intermediate boiler phase and, if desired, a 2-pentene-containing bottom phase are obtained, where steps E b) and E d) can be carried out in a distillation column.

4. A process as claimed in any of claims 1 to 3, wherein the reaction in steps E a) and/or E c) is not complete and a C₂/C₃ low boiler phase, a C₄ intermediate boiler phase and a C_{≥5} bottom phase are obtained in step E b) and/or E d),
where the, if desired combined, low boiler phases are separated into C₂ and C₃ phases by distillation and the C₂ phase is recirculated to step E c),
the, if desired combined, intermediate boiler phases are at least partly recirculated to step E a) and the, if desired combined, bottom phases are at least partly recirculated to step c).

5. A process as claimed in any of claims 1 to 4, wherein, when step A) is carried out, step B is carried out in one stage, and when step A) is not carried out, step B is carried out in two stages, with simultaneous isomerization of 1-butene to 2-butene by bringing the C₄ stream in the liquid phase into contact with a catalyst comprising at least one metal selected from the group consisting of nickel, palladium and platinum on a support, preferably palladium on aluminum oxide, at from 20 to 200°C, a pressure of from 1 to 50 bar, a space velocity of from 0.5 to 30 m³ of fresh feed per m³ of catalyst per hour and a ratio of recycle to input stream of from 0 to 30 at a molar ratio of hydrogen to diolefins of from 0.5 to 50, in order to obtain a C₄ stream.

6. A process as claimed in any of claims 1 to 5, wherein, in step A), the butadiene-selective solvent is selected from the class consisting of polar aprotic solvents, preferably from among acetone, furfural, acetonitrile, dimethylacetamide, dimethylformamide and N-methylpyrrolidone.

7. A process as claimed in any of claims 1 to 6, wherein the etherification of i-butene in step C) is carried out in a reactor cascade using methanol or i-butanol, preferably i-butanol, in the presence of an acid catalyst, preferably an acid ion exchanger, where the reaction mixture flows from the top downward through flooded fixed-bed catalysts in the reactor cascade and the reactor inlet temperature is from 0 to 60°C, preferably from 10 to 50°C, the outlet temperature is from 25 to 85°C, preferably from 35 to 75°C, the pressure is from 2 to 50 bar, preferably from 3 to 20 bar, the ratio of i-butanol to i-butene is from 0.8 to 2.0, preferably from 1.0 to 1.5, and the total conversion corresponds to the equilibrium conversion.

8. A process as claimed in any of claims 1 to 7, wherein step D) is carried out over at least one guard bed comprising high surface area aluminum oxides, silica gels, aluminosilicates and/or molecular sieves, where the purification step can be used for preheating the feed for the subsequent metathesis step.

9. A process as claimed in any of claims 1 to 8, wherein step E) is carried out using a heterogeneous metathesis catalyst comprising a rhenium compound, preferably a rhenium oxide, on an inorganic support.

## Revendications

1. Procédé de préparation d'oléfines à partir de courants en C₄ de vapocraqueurs ou de raffineries, par
A) éventuellement, distillation par extraction de butadiène à partir du courant en C4 avec un solvant sélectif vis-à-vis du butadiène, pour obtenir un courant en C4 fortement appauvri en butadiène,
B) hydrogénation sélective de butadiènes et d'impuretés acétyléniques du courant en C4 de vapocraqueurs ou de raffineries, ou du courant de l'étape A), avec, simultanément ou après-coup, une isomérisation au moins partielle du 1-butène en 2-butène, pour obtenir un courant en C4 qui contienne des n-butènes et de l'isobutène, et qui pour l'essentiel soit exempt de butadiènes et d'impuretés acétyléniques,
C) élimination de l'isobutène du courant en C4 obtenu dans l'étape B), par réaction avec un alcool pour former un éther, qui subit une séparation et éventuellement est redissocié pour donner de l'isobutène pur, dans le but d'obtenir un courant en C4 qui contienne des n-butènes et éventuellement des impuretés de type produits d'oxygénation, et à partir duquel on puisse aussi éliminer par distillation, lors de la séparation de l'éther, les composés à bas et à haut point d'ébullition,
D) utilisation de matériaux adsorbants pour séparer du courant en C4 obtenu dans l'étape C) les impuretés du type produit d'oxygénation,
E) métathèse en deux étapes des butènes du courant en C4 obtenu dans l'étape D), par
a) réaction du 1-butène et du 2-butène contenus dans le courant en C4 pour donner du propène et du 2-pentène en présence d'un catalyseur de métathèse, qui contient au moins un composé d'un métal des groupes secondaires VIb, VIIb ou VIII du Tableau Périodique des Eléments,
b) puis séparation du propène et du 2-pentène formés et des butènes n'ayant pas réagi, et éventuellement élimination au moins partielle d'un ou plusieurs de ces composés,
c) puis réaction du 2-pentène avec de l'éthène pour donner du propène et du 1-butène en présence d'un catalyseur de métathèse qui contient au moins un composé d'un métal des groupes secondaires VIb, VIIb ou VIII du Tableau Périodique des Eléments,
d) puis séparation du propène et du 1-butène formés,
e) éventuellement, extraction au moins partielle d'un courant de 1-butène pur et/ou éventuellement isomérisation au moins partielle du 1-butène formé en 2-butène en présence d'un catalyseur d'isomérisation, puis recyclage du 1-butène non-extrait et du 2-butène éventuellement formé, en même temps que d'une partie de la fraction en C4 de l'étape a) qui n'a pas réagi dans l'étape a),
f) obtention du propène séparé dans les étapes b) et d),
où la séparation de l'étape d) peut être réalisée par conversion du mélange mis à réagir dans l'étape b), puis, dans l'étape e), la fraction en C4 séparée dans l'étape d) et n'ayant pas réagi est éventuellement au moins partiellement extraite, et/ou éventuellement le 1-butène contenu dans cette fraction en C4 est isomérisé au moins partiellement en 2-butène en présence d'un catalyseur d'isomérisation, le mélange obtenu étant ensuite renvoyé dans l'étape a).

2. Procédé selon la revendication 1, dans lequel l'oléfine obtenue est le propène.

3. Procédé selon la revendication 1 ou 2, dans lequel l'étape E b) est une distillation qui peut être réalisée dans une colonne à cloison de séparation, une colonne de rectification primaire ou selon un montage en deux colonnes, où on obtient une phase à bas point de fusion, contenant du propène, éventuellement une phase à point d'ébullition moyen contenant des butènes, et une phase lourde contenant du 2-pentène,
et/ou
où l'étape E d) est une distillation qui peut être mise en oeuvre dans une colonne à cloison de séparation, dans laquelle on obtient une phase à bas point d'ébullition contenant du propène, une phase à point d'ébullition moyen contenant du 1-butène et éventuellement une phase lourde contenant du 2-pentène, les étapes E b) et E d) pouvant être mises en oeuvre dans une colonne de distillation.

4. Procédé selon l'une des revendications 1 à 3, dans lequel la réaction des étapes E a) et/ou E c) n'est pas complète, et, dans l'étape E b) et/ou E d), on obtient une phase à bas point d'ébullition contenant du C2/3, une phase à point d'ébullition moyen contenant du C4 et une phase lourde contenant du C≥5,
où les phases à bas point d'ébullition, éventuellement combinées, sont séparées par distillation en les phases C2 et C3, et la phase C2 est renvoyée dans l'étape E c),
les phases à point d'ébullition moyen, éventuellement combinées sont au moins en partie renvoyées dans l'étape E a), et les phases lourdes éventuellement combinées sont au moins partiellement renvoyées dans l'étape c).

5. Procédé selon l'une des revendications 1 à 4, **caractérisé en ce que** l'étape B) est réalisée en une étape si on met en oeuvre l'étape A), et en deux étapes si l'étape A) n'est pas mise en oeuvre, avec simultanément isomérisation du 1-butène en 2-butène par mise en contact du courant en C4 en phase liquide avec un catalyseur qui contient au moins un métal choisi dans l'ensemble comprenant le nickel, le palladium et le platine sur un support, de préférence le palladium sur oxyde d'aluminium, à une température de 20 à 200°C, sous une pression de 1 à 50 bar, à une vitesse spatiale horaire de 0,5 à 30 m³ de charge fraîche par m³ de catalyseur par heure, et pour un rapport du recyclat au courant d'alimentation de 0 à 30, avec un rapport en moles de l'hydrogène aux dioléfines de 0,5 à 50, pour obtenir un courant en C4.

6. Procédé selon l'une des revendications 1 à 5, **caractérisé en ce que**, dans l'étape A), le solvant sélectif vis-à-vis du butadiène est choisi dans l'ensemble comprenant les solvants polaires aprotiques, de préférence l'acétone, le furfurol, l'acétonitrile, le diméthylacétamide, le diméthylformamide et la N-méthylpyrrolidone.

7. Procédé selon l'une des revendications 1 à 6, **caractérisé en ce que** l'éthérification de l'isobutène de l'étape C) est réalisée dans une cascade de réacteurs avec du méthanol ou de l'isobutanol, de préférence de l'isobutanol en présence d'un catalyseur acide, de préférence d'un échangeur d'ions acide, dans lequel des catalyseurs noyés en lit fixe passent en courant descendant, la température d'entrée du réacteur étant de 0 à 60°C, de préférence de 10 à 50°C, la température de sortie étant de 25 à 85 et de préférence de 35 à 75°C, la pression étant de 2 à 50 bar et de préférence de 3 à 20 bar, et le rapport de l'isobutanol à l'isobutène est de 0,8 à 2,0, de préférence de 1,0 à 1,5, le taux de conversion global correspondant au taux de conversion à l'équilibre.

8. Procédé selon l'une des revendications 1 à 7, **caractérisé en ce que** l'étape D) est mise en oeuvre sur au moins un lit de garde, constitué d'oxydes d'aluminium, de gels de silice, d'aluminosilicates et/ou de tamis moléculaire à grande aire spécifique, l'étape de purification destinée au préchauffage de la charge pouvant être utilisée pour l'étape suivante de métathèse.

9. Procédé selon l'une des revendications 1 à 8, dans lequel on utilise dans l'étape E) un catalyseur hétérogène de métathèse contenant un composé du rhénium, de préférence un oxyde de rhénium sur un support inorganique.
